Europäisches Patentamt

European Patent Office

Office européen des brevets

(11) Veröffentlichungsnummer: **0 480 236 A2**

# EUROPÄISCHE PATENTANMELDUNG

(21) Anmeldenummer: **91116249.3**

(22) Anmeldetag: **24.09.91**

(51) Int. Cl.5: **C12N 15/55**, C12N 15/29, C12N 15/82, C12N 1/21, C12N 5/10, C12Q 1/68

(30) Priorität: **06.10.90 DE 4031758**

(43) Veröffentlichungstag der Anmeldung: **15.04.92 Patentblatt 92/16**

(84) Benannte Vertragsstaaten: **BE CH DE DK ES FR GB IT LI NL SE**

(71) Anmelder: **BAYER AG**

**W-5090 Leverkusen 1 Bayerwerk(DE)**
Anmelder: **Max-Planck-Gesellschaft zur Förderung der Wissenschaften e.V. Bunsenstrasse 10 W-3400 Göttingen(DE)**

(72) Erfinder: **Schreier, Peter, Dr. Dasselstrasse 16 W-5000 Köln(DE)**
Erfinder: **Herget, Thomas, Dr., Imperial Cancer Research Fund Lincoln's Inn Fields London WCA 3PX(GB)**
Erfinder: **Schell, Jeff, Prof. Dr. Carl-von-Linné-Weg 12 W-5000 Köln 30(DE)**

(74) Vertreter: **Schumacher, Günter, Dr. et al c/o Bayer AG Konzernverwaltung RP Patentabteilung W-5090 Leverkusen 1 Bayerwerk(DE)**

(54) **Resistenz-Gene.**

(57) Die vorliegende Erfindung betrifft neue aus Pflanzen isolierte Resistenz-Gene, welche dadurch gekennzeichnet sind, daß sie spezifisch durch Pathogene induzierbar sind und DNA-Sequenzen enthalten, die der cDNA der folgenden Sequenz entsprechen:

1                                                    50
ATCTCGTTCA AGTCGGCGCT CTGGTTGTGG ATGACAGAGC AGAAACCAAA

51                                                  100
ACCTTCTTGC CACAACGTCA TGGTTGGGAA TTACGTGCCA ACAGCATCTG

101                                                 150
ATAGAGCAGC AAATAGAACC TTAGGGTTTG GGTTGGTTAC GAACATCATC

151                 170
AACGGCGGCC TGGACTGCGG

EP 0 480 236 A2

```
1                                                           50
ATCTCGTTCA AGTCGGCGCT CTGGTTGTGG ATGACAGAGC AGAAACCAAA

51                                                          100
ACCTTCTTGC CACAACGTCA TGGTTGGGAA TTACGTGCCA ACAGCATCTG

101                                                         150
ATAGAGCAGC AAATAGAACC TTAGGGTTTG GGTTGGTTAC GAACATCATC

151               170
AACGGCGGCC TGGACTGCGG
```

Die vorliegende Erfindung betrifft neue aus Pflanzen isolierte Resistenz-Gene und ihre Verwendung zur Transformation von Vektoren, Wirtsorganismen und Pflanzen sowie zur Erzeugung von Pflanzen, welche eine erhöhte Resistenz gegenüber Schädlingen aufweisen.

Viele Pflanzen verfügen über natürliche Möglichkeiten zur Abwehr von Schädlingen. Diese Abwehrmaßnahmen werden durch Resistenz-Gene gesteuert. Sie können durch biotische (z.B. Schädlingsangriff) oder abiotische Reize (z.B. UV-Licht) aktiviert werden. Die Mechanismen dieser Resistenzen sind meist noch nicht vollständig aufgeklärt. Es besteht ein Bedürfnis, Resistenz-Gene zur Verfügung zu haben, welche primär durch Schädlinge aktiviert werden und welche in das Genom von Pflanzen eingebaut werden können, um deren bereits vorhandene Resistenz gegenüber Schädlingen weiter zu erhöhen Solche spezifisch durch Schädlinge induzierbare Resistenz-Gene sind noch nicht bekannt geworden.

Es wurden nun neue Resistenz-Gene aus Pflanzen isoliert, welche in die Erbmasse (das Genom) von Pflanzen eingebaut werden können, die keine, unzureichende Mengen oder zu einem verspäteten Zeitpunkt Abwehrstoffe erzeugen, wodurch eine erhöhte Resistenz dieser Pflanzen gegenüber Schädlinge (Pathogene) hervorgerufen werden kann.

Die neuen Resistenz-Gene aus Pflanzen sind dadurch gekennzeichnet, daß sie spezifisch durch Pathogene induzierbar sind und DNA-Sequenzen enthalten, die der cDNA der folgenden Sequenz entsprechen:

```
1                                                        50
ATCTCGTTCA AGTCGGCGCT CTGGTTGTGG ATGACAGAGC AGAAACCAAA

51                                                       100
ACCTTCTTGC CACAACGTCA TGGTTGGGAA TTACGTGCCA ACAGCATCTG

101                                                      150
ATAGAGCAGC AAATAGAACC TTAGGGTTTG GGTTGGTTAC GAACATCATC

151                  170
AACGGCGGCC TGGACTGCGG
```

Es ist überraschend, daß eine neue Art von Resistenz-Genen gefunden werden konnte, die Sequenzen enthalten, die der oben aufgeführten cDNA völlig oder im wesentlichen entsprechen. Diese cDNA-Sequenz ist neu und ihre Existenz konnte nicht vorhergesehen werden.

Weiterhin ist es als überraschend anzusehen, daß die neuen Resistenz-Gene im wesentlichen durch Pathogene (Schädlinge) oder durch Zellwandfragmente von Pathogenen (Elicitoren) aktiviert werden, während übliche andere biotische und abiotische Induktoren ohne eine wesentliche Wirkung bleiben. Die neuen Resistenz-Gene eignen sich daher in einem besonderen Maße zur Erhöhung von Schädlingsresistenzen in transgenen Pflanzen.

Resistenzgene, die DNA-Sequenzen enthalten, welche der oben aufgeführten cDNA entsprechen, enthalten zur cDNA komplementäre DNA-Sequenzen, welche nach der Transkription in mRNA und die Verwendung dieser mRNA als Matrize die aufgeführte cDNA liefern können. Erfindungsgemäße Resistenz-Gene sind Gene, deren Expression spezifisch durch Pathogene induzierbar ist und die mit der oben angegebenen cDNA hybridisieren.

Unter Resistenz-Genen sollen Nukleinsäuren (DNA) verstanden werden, die in Pflanzen die Bildung von Stoffen bewirken, welche geeignet sind, die Pflanzen angreifende Schädlinge abzuwehren, zu vernichten oder ihre Ausbreitung zu behindern. Vorzugsweise handelt es sich bei diesen Stoffen um Enzyme und insbesondere um Hydrolasen, wobei solche Hydrolasen bevorzugt sind, welche $\beta$(1-4)-Verknüpfungen in N-Acetyl-D-glucosamin-Polymeren oder N-Acetyl-D-glucosamin enthaltenden Polymeren spalten können (Chitinasen).

Diese Nukleinsäuren können aus ihrer natürlichen Umgebung (Pflanzen) isoliert vorliegen oder in einen Vektor, z.B. in Plasmide, Phagen oder Cosmide integriert sein oder in einer prokaryontischen, z.B. bakteriellen oder eukaryontischen, z.B. pflanzlichen DNA als "fremde" DNA oder als "zusätzliche" DNA enthalten sein.

Unter Resistenz-Genen sollen auch solche Resistenz-Gene verstanden werden, die an ihrem Anfang und/oder Ende noch DNA-Sequenzen enthalten, die die Funktion der Gene nicht oder nicht wesentlich behindern. Diese auch als "Gen-Einheiten" bezeichneten DNA-Sequenzen entstehen, z.B. durch das Herausschneiden mit Restriktionsenzymen, z.B. mit BamH1, XbaI oder HindIII durch Spaltung der genom-

ischen DNA, wie sie z.B. bei der Extraktion aus der Suspensions-Zell-Linie von Arachis hypogaea (Rolfs, Fritzemeier, Kindl (1981)) erhalten wird.

Die Resistenz-Gene (bzw. die Gen-Einheiten) können in der Form vorliegen, wie sie im Genom von Pflanzen enthalten sind ("genomische" Form, einschließlich nicht kodierender Sequenzen (wie Introns)) oder in einer Form, welche der cDNA ("copy" DNA) entspricht, die über mRNA mit Hilfe von Reverse-Transkriptase/Polymerase erhältlich ist (und keine Introns mehr enthält). Die Resistenz-Gene können auch in teilweise oder vollständig synthetisierter Form vorliegen.

In den erfindungsgemäßen Resistenz-Genen (bzw. den Gen-Einheiten) können DNA-Abschnitte durch im wesentlichen gleichwirkende andere DNA-Abschnitte oder DNA's ersetzt sein, soweit hierdurch die Bildung von im wesentlichen funktionsfähigen Abwehrstoffen nicht gestört oder behindert wird. Auch können sie an den Enden solche DNA Sequenzen tragen, welche für die Handhabung der Gene (bzw. der Gen-Einheiten) jeweils angepaßt sind (z.B. "Linker") .

Im vorliegenden Zusammenhang soll unter "fremder" DNA, solche DNA verstanden werden, welche in einem bestimmten prokaryontischen oder eukaryontischen Genom nicht natürlich vorkommt, sondern erst durch Eingriffe durch den Menschen in dieses Genom aufgenommen wird. "Zusätzliche" DNA soll solche DNA sein, welche in dem jeweiligen prokaryontischen oder eukaryontischen Genom zwar natürlich vorkommt, jedoch in zusätzlicher Menge durch Eingriffe durch den Menschen in dieses Genom aufgenommen wird. Die "fremde" DNA oder "zusätzliche" DNA kann je nach Bedarf und Art des vorliegenden Falles in einem oder mehreren Exemplaren eingebaut werden.

Wie bereits erwähnt, bedeuten Resistenz-Gene vorzugsweise solche Gene, welche Hydrolasen erzeugen, wobei Hydrolasen vom Typ der Chitinasen besonders bevorzugt werden.

Die erfindungsgemäßen Resistenz-Gene stammen aus monokotyledonen (einkeimblättrigen) und dikotyledonen (zweikeimblättrigen) Pflanzen. Als erfindungsgemäße Resistenz-Gene werden solche bevorzugt, welche aus dikotylen (zweikeimblättrigen) Pflanzen, insbesondere Solanaceaen und Leguminosen und besonders bevorzugt aus Erdnuß (Arachis hypogaea) isoliert werden können. Mit Hilfe der oben aufgeführten cDNA als Sonde können Pflanzen nach üblichen Methoden auf das Vorhandensein der Resistenz-Gene untersucht werden.

Als Resistenz Gene werden erfindungsgemäß besonders bevorzugt solche Resistenz-Gene, welche DNA-Sequenzen enthalten, die der oben angegegeben cDNA-Sequenz entsprechen und die durch Pathogene (insbesondere Pilze oder Pilzzellwandfragmente), vorzugsweise durch Phytophthora megasperma oder dessen Zellwandfragmente, induzierbar sind und hieraus insbesondere solche die aus Arachis hypogaea (Erdnuß) isolierbar sind.

Diese Gene bzw. wesentliche Teile davon liegen auf 3 DNA-Fragmenten von ca. 2 bis 3 kbp (im Falle der Spaltung mit Hind III) oder auf Fragmenten zwischen ca. 5 und ca. 23 kbp (im Falle der Spaltung mit XbaI oder BamHI).

Die erfindungsgemäßen Resistenz-Gene können aus Pflanzen, z.B. Erdnuß, unter Verwendung der folgenden cDNA-Sequenz

```
1                                                    50
ATCTCGTTCA AGTCGGCGCT CTGGTTGTGG ATGACAGAGC AGAAACCAAA

51                                                   100
ACCTTCTTGC CACAACGTCA TGGTTGGGAA TTACGTGCCA ACAGCATCTG

101                                                  150
ATAGAGCAGC AAATAGAACC TTAGGGTTTG GGTTGGTTAC GAACATCATC

151            170
AACGGCGGCC TGGACTGCGG
```

als Sonde mit Hilfe der allgemein üblichen und bekannten Verfahren und Methoden der Molekularbiologie aufgefunden, isoliert, gereinigt und nachgewiesen werden.

Man isoliert hierzu die DNA der betreffenden Pflanze und bestimmt mit Hilfe der Southern-Blot Technik günstige Restriktionsendonukleasen (vorzugsweise solche, die Fragmente einheitlicher Größen liefern, wobei die Größe der Fragmente 2 kb nicht unter- und 28 kb nicht überschreiten sollte), mit denen diese DNA verdaut wird. Die enthaltenen Fragmente werden nach ihrer Größe aufgetrennt. Fragmente, die mit der oben aufgeführten cDNA hybridisieren, werden als eine partielle Genbibliothek der jeweiligen Pflanze in geeignete Vektoren kloniert. Mit der Plaque -Hybridisierung werden die Resistenz-Gene in dem entsprechenden Vektor mit Hilfe der cDNA isoliert. Die aus den Isolaten gewonnenen Nukleinsäuren werden zur direkten Transformation von Pflanzen verwendet. Es ist auch möglich, das gewünschte Gen aus den Isolaten mit Hilfe der üblichen Methoden zu isolieren und in anderen Transformationsverfahren, z.B. mit Hilfe von Agrobacterium tumefaciens einzusetzen.

Die mit Hilfe der oben angeführten cDNA isolierten Resistenz-Gene sind im allgemeinen spezifisch durch Pathogene induzierbar. Es sind jedoch Fälle denkbar, in denen, abhängig von der Art der Pflanze, die für die Induktion notwendige Signalkette gestört oder nicht vorhanden ist. Die Resistenz-Induktion kann überprüft werden, indem Pflanzen oder Teile von Pflanzen mit einem Pathogen oder dessen Zellwandfragmenten inkubiert werden. Die spezifische Induzierbarkeit läßt sich mit den üblichen Methoden (z.B. Northern-Analyse) und mit Hilfe der oben angeführten cDNA nachweisen.

Dieses Verfahren soll am Beispiel der Erdnuß Resistenz-Gene, wie folgt, erläutert werden:

Es wird Desoxyribonukleinsäure DNA aus der Suspensions-zell-Linie von Arachis hypogaea (Rolfs et al. 1981) isoliert und je ein Aliquot mit einer Restriktionsendonuklease, vorzugsweise den Enzymen HindIII oder MboI geschnitten. Die DNA wird dann mit Hilfe der Sedimentations-Zentrifugation oder mit Hilfe der Gelelektrophorese nach ihrer Fragmentgröße aufgetrennt (Sambrook, Fritsch und Maniatis, 1989). Die Fraktionen, die Fragmente der Größe bis ca. 10 kb (HindIII) bzw. von ca. 10-20 kb (MboI) enthalten, werden in die Lambda-Vektoren (Lambda ZAP II bzw. Lambda FIX II; Stratagen GmbH, Heidelberg) kloniert. Auf diese Weise werden Teile einer Gen-Bibliothek von Erdnuß angelegt, die Resistenz-Gene im Sinne der Erfindung enthalten. Vektoren, die diese Resistenz-Gene enthalten, lassen sich mit Hilfe der cDNA-Probe, die in pR 3-7 kloniert ist, eindeutig identifizieren und weiter reinigen. Die gereinigte Nukleinsäure, die die Resistenz-Gene enthält, wird dann mit Hilfe der direkten DNA-Transformation oder anderen Transformationsmethoden, vorzugsweise mit Agrobacterium tumefacions, in Tabak oder andere Pflanzen übertragen.

Entsprechend kann bei der Isolierung der Resistenz-Gene aus anderen Pflanzen verfahren werden.

Die oben angegebene cDNA-Sequenz, einschließlich der im wesentlichen gleichwirkenden DNA-Sequenzen (in denen die Verwendbarkeit als Sonde noch erhalten ist), ist Teil der vorliegenden Erfindung. Sie ist nach den bekannten DNA-Synthesemethoden erhältlich oder kann in üblicher Weise aus dem Plasmid pR 3-7 erhalten werden, indem nach Verdauung mit EcoRI oder HindIII ein Fragment von ca. 170 kb isoliert wird. Teil der vorliegenden Erfindung sind auch DNA-Sequenzen, die dieser cDNA entsprechen, welche also auch z.B. nicht kodierende DNA-Sequenzen (Introns) enthalten können, sowie die im wesentlichen gleichwirkenden Sequenzen.

Das Plasmid pR 3-7 besteht aus dem Ausgangsplasmid pUC 19, in dessen Schnittstellen die angegebene cDNA über glatte Enden kloniert wurde. Die cDNA kann z.B. mit EcoRI und HindIII vollständig ausgeschnitten werden, wobei die cDNA durch die unten ersichtlichen Sequenzen am 5'-Ende (Pos-1) und am 3'-Ende flankiert wird (flankierende Enden in Kleinbuchstaben):

```
1
gaattcgagc tcggtacccg gggatcctct agagtcATCT CGTTCAAGTC

51
GGCGCTCTGG TTGTGGATGA GAGAGCAGAA ACCAAAACCT TCTTGCCACA

101
ACGTCATGGT TGGGAATTAC GTGCCAACAG CATCTGATAG AGCAGCAAAT

151
AGAACCTTAG GGTTTGGGTT GGTTACGAAC ATCATCAACG GCGGCCTGGA

201
CTGCGGgacc tgcaggcatg caagctt
```

Diese DNA-Sequenz ist Teil der vorliegenden Erfindung. Sie kann als Probe (wie die cDNA) zum Auffinden erfindungsgemäßer Gene direkt verwendet werden. Falls es gewünscht ist, die cDNA-Sequenz ohne flankierende Sequenzen zu erhalten, kann sie sowohl mit Hilfe der PCR-Reaktion (Polymerase-Ketten-Reaktion) und den synthetischen Oligonukleotiden [5']ATC TCG TTC AAG TCG GCGCT[3'] und [5']CCG CAG TCC AGG CCG CCGTT[3'] oder auch vollständig synthetisch hergestellt werden.

Der Stamm Escherichia coli RG-2, der das Plasmid pR 3-7 enthält, wurde bei der Deutschen Sammlung von Mikroorganismen (DSM), Mascheroder Weg 1b, D-3300 Braunschweig, Bundesrepublik Deutschland in Übereinstimmung mit den Bestimmungen des Budapester Vertrages über die internationale Anerkennung der Hinterlegung von Mikroorganismen für die Zwecke von Patentverfahren hinterlegt und hat die Hinterlegungsnummer DSM 6149 (Hinterlegungsdatum: 28. August 1990).

Dieser Stamm (E. coli K12) sowie seine Mutanten, die das Plasmid enthalten, sind ebenfalls Teil der vorliegenden Erfindung.

Die cDNA (gegebenenfalls mit flankierenden Sequenzen) kann in bekannter Weise durch die Vermehrung des Stammes und anschließende Isolierung leicht nach den bekannten Methoden in den benötigten Mengen gewonnen werden.

Erfindungsgemäße pflanzliche Resistenz-Gene sind solche, die spezifisch durch Pathogene induzierbar sind und DNA-Sequenzen enthalten, die der oben angegebenen cDNA entsprechen. Dies ist so zu verstehen, daß zwischen den DNA-Sequenzen und der cDNA Homologie in unterschiedlichem Ausmaß vorliegt, wobei die Homologie jedoch ausreichend ist, um das Resistenz-Gen mit Hilfe der cDNA zu isolieren.

Die vorliegende Erfindung erstreckt sich somit auch auf solche pflanzliche Resistenzgene, die spezifisch durch Cathogene induziert sind und mit Hilfe der angegebenen cDNA als Sonde isoliert werden können.

Funktionell vollständige Gene, wie die erfindungsgemäßen Resistenz-Gene, bestehen aus regulatorisch wirkenden Teilen (insbesondere dem Promotor) und dem Strukturgen, welches für das jeweilige Protein kodiert.

Beide Genteile können unabhängig voneinander verwendet werden. So ist es möglich, dem regulativ wirkenden Teil eine (vom Resistenz-Gen abweichende) andere DNA-Sequenz nachzuschalten, welche nach dem Einbau in das Pflanzengenom exprimiert werden soll. Da nur wenige isolierte Promotoren bekannt sind und noch keine Promotoren verfügbar waren, welche spezifisch durch Pathogene aktiviert werden und welche ihre Wirkung in Pflanzen entfalten können, stellen die Promotoren der erfindungsgemäßen Resistenz-Gene, welche ebenfalls Teil der vorliegenden Erfindung sind, wertvolle Hilfsmittel bei der Erzeugung transgener Pflanzen mit erhöhter Resistenz gegen Pathogene dar.

Ebenso ist es möglich, den Resistenz-Struktur-Genen einen "fremden" regulatorisch wirkenden Teil vorzuschalten. Dies könnte vorteilhaft sein, wenn bei bestimmten Pflanzen nur bestimmte (z.B. pflanzeneigene) regulatorisch wirkende Gene ausreichend wirksam werden können. Die Resistenz-Struktur-Gene sowie DNA der oben aufgeführten Sequenz (cDNA) stellen somit wertvolle, selbstständig einsetzbare Einheiten dar und sind, wie bereits dargelegt, ebenfalls Teil der vorliegenden Erfindung. Die erfindungsgemäßen Resistenz-Gene können nach den üblichen Methoden in die regulatorisch wirkenden Teile und die Struktur-Gene getrennt werden. Bevorzugt werden die vollständigen erfindungsgemäßen Resistenz-Gene (bzw. die Gen-Einheiten) verwendet.

Mit Hilfe der üblichen Methoden ist es möglich, die Resistenz-Gene (bzw. die Gen-Einheiten) oder ihre Teile ein oder mehrfach (z.B. Tandemanordnung), vorzugsweise einfach, in beliebige prokaryontische (vorzugsweise bakterielle) oder eukaryontische (vorzugsweise pflanzliche) DNA als "fremde" oder "zusätzliche" DNA einzubauen. Die so "modifizierte" rekombinante DNA, welche z.B. zur Transformation von Pflanzen bzw. Pflanzenzellen verwendet werden kann und nach der Transformation in Pflanzen bzw. Pflanzenzellen enthalten ist, ist Bestandteil der vorliegenden Erfindung.

Die Resistenz-Gene (bzw. die Gen-Einheiten) und/oder ihre Teile, die cDNA mit der oben aufgeführten Sequenz und die genomische DNA, welcher dieser cDNA entspricht, sowie die modifizierte DNA können als "fremde" oder "zusätzliche" DNA in Vektoren (insbesondere Plasmiden, Cosmiden oder Phagen), in transformierten Mikroorganismen (vorzugsweise Bakterien, insbesondere Gram-negativen Bakterien, wie E. coli) sowie in transgenen Pflanzenzellen und Pflanzen bzw. in deren DNA enthalten sein. Solche Vektoren, transformierte Mikroorganismen (die auch diese Vektoren enthalten können) sowie die transgenen Pflanzenzellen und Pflanzen und deren DNA stellen Bestandteile der vorliegenden Erfindung dar.

Als Schädlinge, gegen welche mit Hilfe der erfindungsgemäßen Resistenz-Gene Resistenzen, bzw. erhöhte Resistenzen erzielt werden können, kommen vorzugsweise Schädlinge in Frage, welche durch Hydrolasen, insbesondere vom Chitinase-Typ, darin behindert werden, die Pflanzen zu schädigen. Vorzugsweise seien Arthropoden wie Insekten und Milben, Nematoden sowie mikrobielle Schädlinge, wie phytopathogene Pilze und Bakterien genannt. Besonders hervorgehoben werden mikrobielle Schädlinge, insbesondere phytopathogene Pilze.

Zu den schädlichen Insekten gehören insbesondere Insekten der Ordnungen:

Orthoptera, Dermaptera, Isoptera, Thysanoptera, Heteroptera, Homoptera, Lepidoptera, Coleoptera, Hymenoptera und Diptera.

Zu den schädlichen Milben gehören insbesondere: Tarsonemus spp., Panonychus spp. und Tetranychus spp.

Zu den schädlichen Nematoden gehören insbesondere: Pratylenchus spp., Heterodera spp. und Meloidogyne spp.

Zu den mikrobiellen Schädlingen gehören insbesondere die phytopathogenen Pilze:

Plasmodiophoromycetes, Oomycetes, Chytridiomycetes, Zygomycetes, Ascomycetes, Basidiomycetes, Deuteromycetes.

Zu den phytopathogenen Bakterien gehören insbesondere die Pseudomonadaceae, Rhizobiaceae, Enterobacteriaceae, Corynebacteriaceae und Streptomycetaceae.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;

Pseudomonas-Arten, wie beispielsweise Pseudomonas syringae pv. lachrymans;

Erwinia-Arten, wie beispielsweise Erwinia amylovora;

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubense;

Plasmopara-Arten, wie beispielsweise Plasmopara viticola;

Peronospora-Arten, wie beispielsweise Peronospora pisi oder P. brassicae;

Erysiphe-Arten, wie beispielsweise Erysiphe graminis;

Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis;

Pyrenophora-Arten, wie beispielsweise Pyrenophora teres oder P. graminea

(Konidienform: Drechslera, Syn: Helminthosporium);

Cochliobolus-Arten, wie beispielsweise Cochliobolus sativus

(Konidienform: Drechslera, Syn: Helminthosporium);

Uromyces-Arten, wie beispielsweise Uromyces appendiculatus;

Puccinia-Arten, wie beispielsweise Puccinia recondita;

Tilletia-Arten, wie beispielsweise Tilletia caries;

Ustilago-Arten, wie beispielsweise Ustilago nuda oder Ustilago avenae;

Pellicularia-Arten, wie beispielsweise Pellicularia sasakii;

Pyricularia-Arten, wie beispielsweise Pyricularia oryzae;

Fusarium-Arten, wie beispielsweise Fusarium culmorum;

Botrytis-Arten, wie beispielsweise Botrytis cinerea;

Septoria-Arten, wie beispielsweise Septoria nodorum;

Leptosphaeria-Arten, wie beispielsweise Leptosphaeria nodorum;

Cercospora-Arten, wie beispielsweise Cercospora canescens;

Alternaria-Arten, wie beispielsweise Alternaria brassicae;

Pseudocercosporella-Arten, wie beispielwseise Pseudocerco sporella herpotrichoides. Weiterhin sei Helminthosporium carbonum aufgeführt.

Zu den Pflanzen, welchen durch den Einbau der erfindungsgemäßen Resistenz-Gene (bzw. der Gen-Einheiten) Resistenz bzw. eine erhöhte Resistenz gegenüber den obigen Schädlingen verliehen werden kann, (Transformation) gehören praktisch alle Pflanzen. Ein besonderes Bedürfnis zur Resistenzerzeugung besteht naturgemäß bei den Kulturpflanzen, wie Forstpflanzen, z.B. Fichten, Tannen, Douglasien, Kiefern, Lärchen, Buchen und Eichen sowie Nahrungsmittel und Rohstoffe liefernden Pflanzen, z.B. Getreide (insbesondere Weizen, Roggen, Gerste, Hafer, Hirse, Reis und Mais), Kartoffel, Leguminosa wie Hülsenfrüchte und insbesondere Alfalfa, Sojabohnen, Gemüse (insbesondere Kohlarten und Tomaten), Obst (insbesondere Äpfel, Birnen, Kirschen, Weintrauben, Citrus, Ananas und Bananen), Ölpalmen, Tee-, Kakao- und Kaffeesträucher, Tabak, Sisal und Baumwolle sowie bei Heilpflanzen, wie Rauwolfia und Digitalis. Besonders bevorzugt seien Kartoffel, Tomaten, Wein und Leguminosen genannt. Vorzugsweise werden die erfindungsgemäßen Resistenz-Gene als "fremde" DNA in das Genom von Pflanzen eingebaut.

Wie bereits angedeutet, werden erfindungsgemäß die Resistenz-Gene (bzw. die Gen-Einheiten) ein- oder mehrfach (an gleichen oder verschiedenen Stellen des Genoms) in das natürliche pflanzliche Genom, vorzugsweise in das Kern-Genom eingebaut, wobei verschiedene Resistenz-Gene auch miteinander kombiniert werden können.

Bei Pflanzen, welche bereits über erfindungsgemäße Resistenz-Gene verfügen, kann der Einbau eines oder mehrerer erfindungsgemäßer Resistenz-Gene zu einem erheblich verbesserten Resistenzverhalten führen. Gegebenenfalls werden nur die erfindungsgemäßen Strukturgene verwendet, wobei ein evtl. aus der jeweiligen Pflanze isoliertes regulatorisches DNA Element vorgeschaltet wird.

Die erhöhte Resistenz der erfindungsgemäßen transformierten Pflanzenzellen und Pflanzen ist von Bedeutung für Landwirtschaft und Forsten, für den Zierpflanzenanbau, den Heilpflanzenanbau und die Pflanzenzucht.

Die vorliegende Erfindung betrifft somit auch ein Verfahren zur Herstellung transgener Pflanzenzellen (einschließlich Protoplasten) und Pflanzen (einschließlich Pflanzenteile und Samen) mit erhöhter Schädlingsresistenz, welches dadurch gekennzeichnet ist, daß man

(a) ein oder mehrere erfindungsgemäße Resistenz-Gene (bzw. Gen-Einheiten) und/oder Teile der Resistenz-Gene (bzw. der Gen-Einheiten) und/oder erfindungsgemäß modifizierte rekombinante DNA in das Genom von Pflanzenzellen (einschließlich Protoplasten) einsetzt und gegebenenfalls

(b) aus den transformierten Pflanzenzellen (einschließlich Protoplasten) mit einer erhöhten Schädlingsresistenz vollständige transformierte Pflanzen regeneriert und gegebenenfalls

(c) von den so erhaltenen transformierten Pflanzen der Elterngeneration oder weiterer daraus gewonnener Generationen die gewünschten Pflanzenteile (einschließlich Samen) gewinnt.

Die Verfahrensschritte (a), (b) und (c) können nach bekannten Verfahren und Methoden in üblicher Weise durchgeführt werden.

Transgene Pflanzenzellen (einschließlich Protoplasten) und Pflanzen (einschließlich Pflanzenteile und Samen), welche ein oder mehrere erfindungsgemäße Resistenz-Gene (bzw. Gen-Einheiten) und/oder Teile der Resistenz-Gene (bzw. der Gen-Einheiten) als "fremde" oder "zusätzliche" DNA enthalten sowie solche transgene Pflanzenzellen und Pflanzen, welche nach den obigen Verfahren erhältlich sind, gehören ebenfalls zur vorliegenden Erfindung.

Teile der vorliegenden Erfindung sind auch die:

(a) Verwendung der erfindungsgemäßen Resistenz-Gene (bzw. der Gen-Einheiten) und/oder ihrer Teile und/oder der erfindungsgemäß modifizierten DNA und/oder der erfindungsgemäßen Vektoren und/oder der erfindungsgemäßen transformierten Mikroorganismen zur Erzeugung von transgenen Pflanzenzellen (einschließlich Protoplasten) und Pflanzen (einschließlich Pflanzenteilen und Samen) sowie die

(b) Verwendung der erfindungsgemäßen transgenen Pflanzenzellen (einschließlich Protoplasten) und Pflanzen (einschließlich Pflanzenteilen und Samen) zur Erzeugung von Vermehrungsmaterial sowie zur Erzeugung neuer transgener Pflanzen und deren Vermehrungsmaterial und allgemein die

(c) Verwendung der erfindungsgemäßen Resistenz-Gene (bzw. der Gen-Einheiten) und/oder ihrer Teile und/oder der erfindungsgemäß modifizierten DNA in transgenen Pflanzen zur Bekämpfung von Schädlingen.

Eine Anzahl verschiedener Methoden steht zur Verfügung, die Resistenz-Gene bzw. die Gen-Einheiten oder ihre Teile als "fremde" oder "zusätzliche" DNA in das genetische Material von Pflanzen bzw. Pflanzenzellen einzusetzen. Der Gentransfer kann nach den allgemein üblichen bekannten Methoden erfolgen, wobei der Fachmann die jeweils geeignete Methode ohne Schwierigkeiten ermitteln kann.

Das Ti-Plasmid von Agrobacterium tumefaciens steht als besonders günstiger und breit einsetzbarer Vektor zur Übertragung von fremder DNA in Genome dikotyler und monokotyler, vorzugsweise dikotyler Pflanzen zur Verfügung. Die Resistenz-Gene oder ihre Teile werden in die T-DNA von geeigneten Ti-Plasmiden eingesetzt (z.B. Zambryski et al. 1983) und durch Infektion der Pflanze, Infektion von Pflanzenteilen oder Pflanzengeweben, wie z.B. von Blattscheiben, Stengeln, Hypokotylen, Kotyledonen, Meristemen und davon ableitenden Geweben, wie z.B. sekundären Embryonen und Kalli oder durch Kokultur von Protoplasten mit Agrobacterium tumefaciens übertragen.

Eine Alternative ist die Inkubation von gereinigter DNA, die das gewünschte Gen enthält in Pflanzenprotoplasten (z.B. Hain et al., 1985; Krens et al., 1982; Paszkowski et al., 1984) in Gegenwart von Polykationen oder Calziumsalzen und Polyethylenglykol.

Die DNA-Aufnahme kann auch zusätzlich durch ein elektrisches Feld (Elektroporation) begünstigt werden (z.B. Fromm et al. 1986).

Die DNA kann in bekannter Weise auch über Pflanzenpollen eingeführt werden, indem Pollen mit physikalisch beschleunigten Partikeln "beschossen" werden, welche die DNA tragen (vgl. EP-A 0 270 356).

Die Regeneration der Pflanzen erfolgt in bekannter Weise mit Hilfe geeigneter Nährmedien (z.B. Nagy und Maliga 1976).

In einer bevorzugten Ausführungsform des erfindungsgemäßen Verfahrens (gemäß der Methode aus EP-A 116 718) werden die erfindungsgemäßen Gene bzw. Geneinheiten in einen geeigneten intermediaeren E.coli Vektor z.B. pGV700 oder pGV710, (vergl. EP-A-116 718; Deblaere et al. 1986) bzw. vorzugsweise Derivaten davon, die zusätzlich ein Reportergen wie z.B. nptII (Herrera-Estrella et al. 1983) oder hpt (Van den Elzen et al. 1986) enthalten, kloniert.

Der Escherichia coli Stamm AZ 4, der den Vektor pGV710 in leicht isolierbarer Form enthält, wurde bei der Deutschen Sammlung von Mikroorganismen (DSM), Grisebachstraße 8, D-3400 Göttingen, Bundesrepublik Deutschland in Übereinstimmung mit den Bestimmungen des Budapester Vertrages über die internationale Anerkennung der Hinterlegung von Mikroorganismen für die Zwecke von Patentverfahren hinterlegt und hat die Hinterlegungsnummer DSM 3164.

Das so konstruierte Plasmid wird auf Agrobacterium tumefaciens, das z.B. pGV 3850 bzw. Derivate davon enthält (Zambryski et al. 1983) mit üblichen Methoden (z.B. Van Haute et al. 1983) übertragen. Alternativ dazu kann die Resistenz-Geneinheit in einem binaeren Vektor (z.B. Koncz und Schell 1986) kloniert und wie oben beschrieben in einen geeigneten Agrobakterium Stamm (Koncz und Schell 1986) transferiert werden. Der resultierende Agrobakterium Stamm, der die Resistenz-Geneinheit in einer auf Pflanzen transferierbaren Form enthält wird im weiteren zur Pflanzentransformation verwendet.

In einer weiteren bevorzugten Ausführungsform wird ein geeignetes Plasmid mit dem Resistenz-Gen, gegebenenfalls zusammen mit einem anderen Plasmid, das ein Reportergen für Pflanzenzellen, z.B. für Kanamycin-Resistenz (z.B. Herrera-Estrella et al. 1983) oder eine Hydromycin-Resistenz (van den Elzen, 1986) enthält, vorzugsweise pLGV neo 2103 (Hain et al. 1985), pLGV 23 neo (Herrera-Estrella 1983), pMON 129 (Fraley R.T. et al., (1983)), pAK 1003, pAK 2004 (Velten J. et al. (1984)) oder pGSST neo 3 (pGSST3) (EP-A-189 707), in üblicher Weise durch direkten Gentransfer auf Pflanzenprotoplasten übertragen (z.B. Hain et al 1985). Dabei können das bzw. die Plasmide in zirkulärer, vorzugsweise jedoch in linearer Form, vorliegen. Bei der Verwendung eines Plasmids mit Reportergen werden Kanamycin-resistente Protoplasten dann auf Expression von Resistenz-Faktoren überprüft. Im anderen Fall (ohne Reportergen) werden die resultierenden Kalli auf die Expression des Resistenz-Gens geprüft (Screening mit üblichen Methoden).

Transformierte (transgene) Pflanzen bzw. Pflanzenzellen werden nach den bekannten Methoden, z.B. durch Blattscheiben Transformation (z.B. Horsch et al. 1985), durch Cokultur regenerierender Pflanzenprotoplasten oder Zellkulturen mit Agrobacterium tumefaciens (z.B. Marton et al. 1979, Hain et al. 1985) oder durch direkte DNA Transfektion erzeugt. Resultierende transformierte Pflanzen werden entweder durch Selektion auf die Expression des Reportergens, z.B. durch die Phosphorylierung von Kanamycin-sulfat in vitro (Reis et al. 1984; Schreier et al. 1985) oder durch die Expression der Nopalinsynthase (nach Aerts et al. 1983) nachgewiesen.

Die Resistenz-Faktoren, z.B. die Chitinase können auch in bekannter Weise mit Hilfe spezifischer Antikörper in transformierten Pflanzen nachgewiesen werden.

Die Kultivierung der transformierten Pflanzenzellen sowie die Regeneration zu vollständigen Pflanzen erfolgt nach den allgemein üblichen Methoden mit Hilfe der jeweils geeigneten Nährmedien.

Sowohl die transgenen Pflanzenzellen als auch die transgenen Pflanzen, welche erfindungsgemäße Resistenz-Gene bzw. Gen-Einheiten enthalten und welche Bestandteile der vorliegenden Erfindung sind, zeigen eine erheblich höhere Resistenz gegen Schädlinge, insbesondere pflanzenpathogene Pilze.

Im Zusammenhang mit der vorliegenden Erfindung bedeutet der Ausdruck "Pflanzen" sowohl vollständige Pflanzen als auch Pflanzenteile, wie Blätter, Samen, Knollen, Stecklinge u.s.w.. "Pflanzenzellen" schließen Protoplasten, Zelllinien, Pflanzenkalli usw. ein. "Vermehrungsmaterial" bedeutet Pflanzen (wie oben definiert) und Pflanzenzellen (wie oben definiert), welche zur Vermehrung der transgenen Pflanzen und Pflanzenzellen verwendet werden können. Vermehrungsmaterial der erfindungsgemäßen transgenen Pflanzen ist ebenfalls Teil der vorliegenden Erfindung.

Die vorliegende Erfindung schließt auch Gene und Genteile sowie DNA-Sequenzen mit "im wesentlichen gleichwirkenden DNA-Sequenzen" ein. Die Gene, Genteile und DNA-Sequenzen können in ihrer genomischen Form vorliegen und z.B. auch nicht codierende Teile, wie Introns, enthalten. Sie können auch ganz oder teilweise in chemisch synthetisierter Form vorliegen.

Im vorliegenden Zusammenhang bedeutet der Ausdruck "im wesentlichen gleichwirkende DNA-Sequenzen", daß die Erfindung auch solche Modifikationen umfaßt, bei welchen die Funktion der Resistenz-Gene und ihrer Teile nicht derart beeinträchtigt ist, daß die Resistenz-Faktoren nicht mehr gebildet werden oder der regulatorische Genteil nicht mehr wirksam wird. Bei der erfindungsgemäßen cDNA bedeutet "im wesentlichen gleichwirkende DNA-Sequenzen" Modifikationen, die die erfindungsgemäße Verwendung der cDNA als Sonde zum Auffinden der erfindungsgemäßen Gene nicht wesentlich beeinträchtigen. Entsprechende Modifikationen können durch den Ersatz, die Hinzufügung und/oder die Entfernung von DNA-Abschnitten, einzelner Kodons und/oder einzelner Nukleinsäuren erfolgen.

Bei den erfindungsgemäß verwendbaren Mikroorganismen bedeutet "Mutanten" solche modifizierten Mikroorganismen, welche noch die für die Ausführung der Erfindung wesentlichen Merkmale aufweisen, insbesondere die jeweiligen Plasmide enthalten.

Die vorliegende Erfindung soll anhand der folgenden beispielhaften Ausführungen näher erläutert werden:

1. Isolierung von Resistenz-Genen aus Arachis hypogaea

Es wird Desoxyribonukleinsäure DNA aus der Suspensionszell-Linie von Arachis hypogaea (Rolfs et al. 1981) isoliert und je ein Aliquot mit einer Restriktionsendonuklease vorzugsweise den Enzymen HindIII oder MboI geschnitten. Die DNA wird dann mit Hilfe der Sedimentations-Zentrifugation oder mit Hilfe der Gelelektrophorese nach ihrer Fragmentgröße aufgetrennt (Sambrook, Fritsch und Maniatis, 1989, Molecular Cloning; A Laboratory Manual 2nd Ed., Cold Spring Harbor Laboratory Press USA). Im Falle einer Zentrifugation, werden die Fragmente auf einem vorgeformten Gradienten von 30-10 % K-Acetat-Lösung in 10mM Tris-HCl pH 7,5 aufgetrennt. Um Diffusion der Fragmente so weit wie möglich zu unterdrücken, wird die Zentrifugation bei höchstmöglicher Geschwindigkeit in einer präparativen Ultrazentrifuge durchgeführt. Es werden jeweils nicht mehr als 100 $\mu$g geschnittene DNA in einem Volumen $\leq$ 100 $\mu$l auf einem Gradienten (Gradienten-Volumen ca. 13 ml) getrennt. Die Größe der nach der Zentrifugation gewählten Fraktionen wird je nach dem gewünschten Auflösungsvermögen gewählt (z.B. je 200 $\mu$l). Die Fraktionen, die Fragmente der Größe bis ca. 10 kb (HindIII) bzw. von 10-20 kb (MboI) enthalten, werden in die Lambda-Vektoren (Lambda ZAP II bzw. Lambda FIX II; Stratagen GmbH, Heidelberg) kloniert. Als Beispiel für das Erzeugen einer partiellen Genbibliothek, sei die Klonierung der HindIII Fragmente in Lambda ZAP II erläutert. Der käufliche Vektor wird mit SpeI verdaut und die entstehenden klebrigen Enden:

**A**
**TGATC**

durch partielles Auffüllen mit C und T in

**ACT**
**TGATC**

überführt,
ebenso werden die HindIII Enden der zu klonierenden Fragmente behandelt, d.h.:

**A**
**TTCGA**

wird mit A und G in

**AAG**
**TTCGA**

überführt.

Auf diese Weise entstehen Enden, die trotz unterschiedlicher Herkunft miteinander in herkömmlicher Art und Weise verklebt werden können. Da in den Vektor Lambda ZAP II bevorzugt Fragmente bis zu 10 kbp kloniert werden können, werden hierbei erneut große Fragmente diskriminiert. Auf diese Weise werden jeweils Teile einer Gen-Bibliothek von Erdnuß angelegt, die die Resistenz-Gene im Sinne der Erfindung enthalten. Vektoren, die diese Resistenz-Gene enthalten, lassen sich mit Hilfe der cDNA-probe, die in pR 3-7 kloniert ist, eindeutig identifizieren und weiter reinigen. Die gereinigte Nukleinsäure, die die Resistenzgene enthält, wird dann mit Hilfe der direkten DNA-Transformation unter Mitwirkung von Polyethylenglykol (PEG) oder einer Partikel Schießanlage sowie anderen Transformationsmethoden wie z.B. mit Hilfe von Agrobakterien in Tabak oder andere Pflanzen übertragen.

2. Transformation von Tabak

a) Kultur von Tabaksprossen und Isolierung von Tabakprotoplasten:
Nicotiana tabacum (Petit Havana SR1) wird als sterile Sproßkultur auf hormonfreiem LS Medium (Linsmaier und Skoog 1965) vermehrt. In Abständen von ca. 6-8 Wochen werden Sproßabschnitte auf frisches LS-Medium umgesetzt. Die Sproßkulturen werden bei 12 h Licht (1000-3000 Lux) in einem Kulturraum bei 24-26°C gehalten.

Für die Isolierung von Blattprotoplasten werden ca. 2 g Blätter (ca. 3-5 cm lang) mit einer frischen Rasierklinge in kleine Stücke (0,5 cm x 1 cm) geschnitten. Das Blattmaterial wird in 20 ml Enzymlösung, bestehend aus K3 Medium (Nagy und Maliga 1976), 0,4 m Saccharose, pH 5,6, 2 % Zellulase R10 (Serva), 0,5 % Macerozym R10 (Serva) für 14-16 h bei Raumtemperatur inkubiert. Danach werden die Protoplasten durch Filtration über 0,30 mm und 0,1 mm Stahlsiebe von Zellresten getrennt. Das Filtrat wird 10 Minuten lang bei 100 x g zentrifugiert. Während dieser Zentrifugation flotieren intakte Protoplasten und sammeln sich in einer Bande am oberen Rand der Enzymlösung. Das Pellet aus Zellresten und die Enzymlösung werden mit einer Glaskapillare abgesaugt. Die vorgereinigten Protoplasten werden mit frischem K3 Medium (0,4 M Saccharose als Osmotikum) auf 10 ml aufgefüllt und erneut flotiert. Das Waschmedium wird abgesaugt und die Protoplasten werden für Kultur oder folgende Infektion mit Agrobakterien (Kokultur) auf 1-2 x $10^5$/ml verdünnt. Die Protoplastenkonzentration wird in einer Zählkammer bestimmt.

b) Transformation von regenerierenden Tabakprotoplasten durch Kokultur mit Agrobacterium tumefaciens:

Es wird im folgenden die Methode von Marton et al. 1979 mit kleinen Veränderungen benutzt. Die Protoplasten werden wie beschrieben isoliert und in einer Dichte von 1-2 x $10^5$/ml in K3 Medium (0,4 m Saccharose, 0,1 mg/l NAA, 0,2 ml in K3 Medium (0,4 m Saccharose, 0,1 mg/l NAA, 0,2 mg Kinetin)) 2 Tage im Dunkeln und ein bis zwei Tage lang unter Schwachlicht (500 lux) bei 26°C inkubiert. Sobald die ersten Teilungen der Protoplasten auftreten, werden 30 $\mu$l einer Agrobakteriumsuspension in minimal A (Am) Medium (Dichte ca. $10^9$ Agrobakterien/ml) zu 3 ml regenerierenden Protoplasten gegeben. Die Kokulturdauer beträgt 3-4 Tage bei 20°C im Dunkeln. Danach werden die Tabakzellen in 12 ml Zentrifugenrohrchen gefüllt, mit Seewasser (600 mOsm/kg) auf 10 ml verdünnt und bei 60 x g 10 Minuten lang pelletiert. Dieser Waschvorgang wird noch 1-2 x wiederholt um den größten Teil der Agrobakterien zu entfernen. Die Zellsuspension wird in einer Dichte von 5 x $10^4$/ml in K3 Medium (0,3 m Saccharose) mit 1 mg/l NAA (Naphthyl-1-essigsäure), 0,2 mg/l Kinetin und 500 mg/l des Cephalosporin-Antibiotikums Cefotaxim kultiviert. Die Zellsuspension wird jede Woche mit frischem K3 Medium verdünnt und der osmotische Wert des Mediums graduell um 0,05 m Saccharose (ca. 60 mOsm/kg) pro Woche reduziert. Die Selektion mit Kanamycin (100 mg/l Kanamycinsulfat (Sigma), 660 mg/g aktives Km) wird 2-3 Wochen nach der Kokultur in Agarose Bead Typ Kultur (Shillito et al. 1983) gestartet. Kanamycinresistente Kolonien können 3-4 Wochen nach Beginn der Selektion vom Hintergrund zurückgebliebener Kolonien unterschieden werden.

c) Direkte Transformation von Tabakprotoplasten mit DNA. Calciumnitrat-PEG Transformation.

In einer Petrischale werden ca. $10^6$ Protoplasten in 180 $\mu$l K3 Medium mit 20 $\mu$l wäßriger DNA Lösung welche 0,5 $\mu$g/$\mu$l der DNA, die das Resistenz-Gen enthält, und 0,5 $\mu$g/$\mu$l pLGV neo 2103 (Hain et al. 1985) enthält, vorsichtig gemischt. Anschließend werden 200 $\mu$l Fusionslösung (0,1 m Calciumnitrat, 0,45 M Mannit, 25 % Polyethylenglykol (PEG 6000), pH 9) vorsichtig zugegeben. Nach 15 Minuten werden 5 ml Waschlösung (0,275 M Calciumnitrat pH 6) addiert und nach weiteren 5 Minuten werden die Protoplasten in ein Zentrifugenröhrchen transferiert und bei 60 x g pelliert. Das Pellet wird in einer kleinen Menge K3 Medium aufgenommen und wie im nächsten Abschnitt beschrieben kultiviert, Alternativ können die Protoplasten nach Hain et al. 1985 tranformiert werden.

Die Transformation kann auch ohne den Zusatz der 0,5 $\mu$g/$\mu$l pLGV neo 2103 durchgeführt werden. Da in diesem Fall kein Reportergen eingesetzt wird, werden die resultierenden Kalli auf das Vorhandensein des Resistenz-Gens mit Hilfe einer Dot-Blot-Hybridisierung überprüft. Als Hybridisierungs-Probe ist die oben angegebene cDNA aus pR 3-7 verwendbar. Selbstverständlich können auch andere Nachweismethoden, wie Test mit Antikörpern eingesetzt werden.

d) Kultur der mit DNA inkubierten Protoplasten und Selektion Kanamycin resistenter Kalli:

Für die im folgenden beschriebene Kultur und Selektion Kanamycin resistenter Kolonien wird eine modifizierte "Bead Type culture"-Technik (Shillito et al. 1983) verwendet. Eine Woche nach Behandlung der Protoplasten mit DNA (vgl. c) werden 3 ml der Zellsuspension mit 3 ml K3 Medium (0,3 M Saccharose + Hormone; 1,2 % (Seaplaque) LMT Agarose (low melting agarose, Marine Colloids) in 5 cm Petrischalen gemischt. Für diesen Zweck wird Agarose trocken autoklaviert und nach Zugabe von K3 Medium im Mikrowellenherd kurz aufgekocht. Nach Erstarren der Agarose werden die Agarosescheiben ("beads") mit den eingebetteten Tabakmikrokalli für weitere Kultur und Selektion in 10 cm Petrischalen transferiert und je 10 ml K3 Medium (0,3 M Saccharose, 1 mg/l NAA, 0,2 mg/l Kinetin) und 100 mg/l Kanamycinsulfat (Sigma) addiert. Das Flüssigmedium wird jede Woche gewechselt. Dabei wird der osmotische Wert des Mediums stufenweise herabgesetzt.

Pro Woche wird das Austauschmedium (K3 + Km) um 0,05 m an Saccharose (ca. 60 mOsm) reduziert.

Schema der Selektion kanamycinresistenter Tabakkolonien nach DNA Transformation:

```
    0,4 M  0,3 M  0,25 M  0,20 M  0,15M  0,10 M  Saccha-
                                                  rose im
                                                  Flüssig-
                                                  medium

A    E S                                  K
    _____

     1       2       3       4      5      6  Wochen
                                                nach DNA
                                                Aufnahme

      (K3 Medium 1 mg NAA, 0,2 mg Kinetin)


A = DNA Aufnahme

E = Einbettung in Agarose

S = Selektion mit Kanamycin (100 mg/l Kanamycinsulfat)

K = Kanamycinresistente Kolonien können vom Hintergrund
      eindeutig unterschieden werden
```

e) Regeneration kanamycinresistenter Pflanzen:

Sobald die kanamycinresistenten Kolonien einen Durchmesser von ca. 0,5 cm erreicht haben, wird die Hälfte auf Regenerationsmedium (LS-Medium, 2 % Saccharose, 0,5 mg/l Benzylaminopurin BAP) gesetzt und bei 12 h Licht (3000-5000 lux) und 24°C im Kulturraum gehalten. Die andere Hälfte wird als Kalluskultur auf LS Medium mit 1 mg/l NAA, 0,2 mg/l Kinetin, 0,1 mg/l BAP und 100 mg/l Kanamycinsulfat propagiert. Wenn die regenerierten Sproße ca. 1 cm groß sind, werden sie abgeschnitten und auf 1/2 LS Medium (1 % Saccharose, 0,8 % Agar) ohne Wachstumsregulatoren zur Bewurzelung gesetzt. Die Sproße werden auf 1/2 MS-Medium mit 100 mg/l Kanamycinsulfat bewurzelt und später in Erde umgesetzt.

f) Transformation von Blattscheiben durch Agrobacterium tumefaciens

Für die Transformation von Blattscheiben (Horsch et al. 1985) werden ca. 2-3 cm lange Blätter von sterilen Sproßkulturen in Scheiben von 1 cm Durchmesser gestanzt und mit einer Suspension entsprechender Agrobacterien (ca. $10^9$/ml) (vgl. b) in Am-Medium siehe unten) für ca. 5 Minuten inkubiert. Die infizierten Blattstücke werden auf MS-Medium (siehe unten) ohne Hormone für 3-4 Tage bei ca. 24°C gehalten. Während dieser Zeit überwächst Agrobakterium die Blattstücke. Die Blattstücke werden anschließend in MS-Medium (0,5 mg/ml BAP, 0,1 mg/ml NAA) gewaschen und auf das gleiche Medium (0,8 % Agar) mit 500 $\mu$g/ml Cefotaxim und 100 $\mu$g/ml Kanamycinsulfat (Sigma) gelegt. Nach zwei Wochen sollte das Medium erneuert werden. Transformierte Sproße werden nach weiteren 2-3 Wochen sichtbar. Die Regeneration von Sproßen sollte parallel auch ohne Selektionsdruck durchgeführt werden. Die regenerierten Sproße müssen dann durch biologische Tests z.B. auf Nopalinsynthase oder Resistenz-Gen-Aktivität auf Transformation getestet weren. Auf diese Weise werden 1-10 % transformierte Sproße erhalten.

g) Biochemische Nachweismethode der Transformation

Nachweis von Nopalin in Pflanzengeweben:

Nopalin wird wie bei Otten und Schilperoort (1978) und Aerts et al. (1979) beschrieben, wie folgt, nachgewiesen. 50 mg Pflanzenmaterial (Kallus oder Blattstücke) werden über Nacht in LS Medium mit 0,1 M Arginin bei Raumtemperatur in einem Eppendorfgefäß inkubiert. Das Pflanzenmaterial wird danach auf saugfähigem Papier abgetupft, in einem frischen Eppendorfzentrifugengefäß mit einem Glasstab homogenisiert und 2 Min. in einer Eppendorfzentrifuge zentrifugiert. 2 $\mu$l des Überstandes werden auf ein für Elektrophorese geeignetes Papier (Whatman 3 MM Papier) (20 x 40 cm) punktförmig aufgetragen und getrocknet. Das Papier wird mit dem Laufmittel (5 % Ameisensäure, 15 % Essigsäure, 80 % $H_2O$, pH 1,8) getränkt und bei 400 V für 45 Minuten elektrophoretisiert. Nopalin läuft zur Kathode hin. Das Papier wird dann mit einem Luftstrom heiß getrocknet und durch Phenanthrenchinon-Färbemittel (gleiches Volumen 0,02 % Phenanthrenchinon in Ethanol und 10 % NaOH in 60 % Ethanol) in Laufrichtung gezogen. Das getrocknete Papier wird unter langwelligem UV-Licht betrachtet und fotografiert. Arginin und Argininderivate werden mit dem Reagenz gelb fluoreszierend gefärbt.

Neomycin-Phosphotransferase (NPT II) Enzymtest:

NPT II Aktivität in Pflanzengewebe wird durch in situ Phosphorylierung von Kanamycin, wie bei Reiß et al. (1984) beschrieben und von Schreier et al. (1985) modifiziert, wie folgt, nachgewiesen. 50 mg Pflanzengewebe werden in 50 $\mu$l Extraktionspuffer (10 % Glycerin, 5 % 2-Mercaptoethanol, 0,1 % SDS, 0,025 % Bromphenolblau, 62,5 mM Tris pH 6,8) unter Zusatz von Glaspulver auf Eis homogenisiert und 10 Minuten lang in einer Eppendorfzentrifuge bei 4°C zentrifugiert. 50 $\mu$l des Überstandes werden auf ein natives Polyacrylamidgel (145 x 110 x 1,2 mm; Trenngel: 10 % Acrylamid, 0,33 % Bisacrylamid, 0,375 M Tris pH 8,8, Sammelgel: 5 % Acrylamid, 0,165 % Bisacrylamid, 0,125 M Tris pH 6,8) aufgetragen und über Nacht bei 4°C und 60 V elektrophoretisiert. Sobald der Bromphenolblau-Marker aus dem Gel herausläuft, wird das Gel zweimal mit destilliertem Wasser 10 Min. lang und einmal 30 Min. mit Reaktionspuffer gewaschen (67 mM Tris-Maleat, pH 7,1, 42 mM $MgCl_2$, 400 mM Ammoniumchlorid). Das Gel wird auf eine gleichgroße Glasplatte gelegt und mit 40 ml 1 %iger Agarose in Reaktionspuffer, der die Substrate Kanamycinsulfat (20 $\mu$g/ml) und 20-200 $\mu$Ci $^{32}$P ATP (Amersham) enthält, überschichtet. Das Sandwichgel wird 30 Min. bei Zimmertemperatur inkubiert und dann wird ein Blatt Phosphozellulosepapier P81 (Whatman) auf die Agarose gelegt. Darüber werden vier Filtrierpapierlagen 3 MM, (Whatman) und einige Papierhandtücher gestapelt. Der Transfer von in situ phosphoryliertem radioaktiven Kanamycinphosphat auf das P81 Papier wird nach 3-4 h gestoppt. Das P81 Papier wird für 30 min. in einer Lösung von Proteinase K und 1 % Natriumdodecyl sulfat (SDS) bei 60°C inkubiert und dann 3-4 mal in 250 ml 10 mM Phosphatpuffer pH 7,5 bei 80°C gewaschen, getrocknet und für 1-12 h lang bei -70°C autoradiografiert (XAR5 Film Kodak).

### 3. Transformation von Medicago sativa (Luzerne)

a) Pflanzenmaterial

Die Pflanze Medicago sativa (Regen S, Klon $RA_3$ Walker et al, 1978) wird als sterile Sproßkultur auf LS Medium (Linsmaier und Skoog, 1965), im Langtag (16 h Licht, 8 h Dunkelheit) bei 26 ± 2°C kultiviert.

b) Kulturbedingungen

Als Kulturgefäße für Sproßkulturen dienen Glasgefäße (250 ml - 1,5 l) mit lose aufliegenden Glasdeckeln. Für alle anderen Pflanzenkulturen (Embryo, Kallus, Protoplasten) werden Petrischalen aus Plastik benutzt.

Die Pflanzen und Gewebekulturen bis auf die Protoplasten werden in Kulturräumen im Langtag (16 h Licht, 8 h Dunkelheit) bei 26 ± 2°C kultiviert. Die Leuchtstoffröhren haben die Lichtfarbe Universal Weiß (Osram L58W/25). Der Abstand der Röhren von den Kulturen beträgt 10-30 cm, das entspricht 1500-4500 Lux Lichtintensität. Die Luftfeuchtigkeit bleibt unreguliert. Die Protoplasten werden in Kulturschränken bei maximal 500 Lux und 26°C kultiviert.

c) Kalluskultur

Kallus wird von Petiolen der Gewächshauspflanzen induziert. Ca. 5 cm lange Petiolenstücke werden von den Gewächshauspflanzen mit einem Skalpell abgeschnitten. Die Petiolenstücke werden zuerst oberflächensterilisiert:

- 1 min in 70% Ethanol
- 10 min in 10% handelsüblichem Desinfektionsmittel (z.B. Dan Klorix)
- 3 Waschungen in sterilem Leitungswasser.

Nach der Sterilisation werden die Petiolenstücke in 1-1,5 cm lange Stücke geschnitten und in Petrischalen auf festes Agarmedium ausgelegt. Drei verschiedene Medien werden zur Kallusinduktion und Weiterkultur benutzt:

1. $B_5$h (Atanassov und Brown, 1984)

2. SHR: SH (Shenk und Hildebrandt, 1972) mit 25 $\mu$M (4,655 mg/l) NAA und 10 $\mu$M (2,15 mg/l) Kinetin (Walker und Sato, 1981)

3. $B_5H_3$: $B_5$ (Gamborg et al., 1968) mit 2,6 $\mu$M (0,5 mg/l) NAA, 2,2 $\mu$M (0,5 mg/l) BAP, 2,2 $\mu$M (0,5 mg/1) 2,4D (Oelck, Dissertation 1984).

Nach drei Wochen werden jeweils die äußeren Teile des Kallus mit einem Skalpell abgeschnitten und auf frischem Medium subkultiviert.

d) Kallusregeneration

Die Regeneration von Pflanzen aus Kallus wird nach dem Protokoll von Stuart und Strickland, 1984 a, b, mit Modifikationen durchgeführt.

Die somatische Embryogenese wird durch eine Inkubation von Kallusgewebe in flüssigem SH-Medium (Shenk und Hildebrandt, 1972), das 50 $\mu$M (11 mg/l) 2,4D und 5 $\mu$M (1,07 mg/l) Kinetin enthält, induziert. In einem Erlenmeyerkolben (100 ml in einem 500 ml Kolben) werden pro ml Medium 30 mg Kallus (Frischgewicht) zugegeben. Die Induktion erfolgt für 3-4 Tage auf einem Schüttler (100 Upm) bei 26°C im Pflanzenkulturraum. Anschließend wird das Kallusgewebe vom Medium auf einem Sieb (850 $\mu$m$^2$) getrennt.

Mit einem Spatel wird es durch das Sieb gepreßt und kleine Zellhaufen werden auf einem sich darunter befindenden Sieb mit der Maschenweite 250 $\mu$m$^2$ gesammelt. Pro 100 ml Induktionsmedium werden die Zellhaufen mit 500 ml SHJ-Medium ohne Hormone (SH) gewaschen. Die Waschlösung wird durch Abtropfen so weit wie möglich entfernt (ca. 5 min). Das Frischgewicht wird bestimmt und die Zellhaufen werden in SH-Medium resuspendiert. 75 mg in 0,5 ml werden in einer Pipette auf ca. 10 ml festes Regenerationsmedium SHR aufgebracht. Das Regenerationsmedium SHR besteht aus SH-Medium mit 25 mM NH$_4^+$ und 100 mM L-Prolin in 3% Saccharose.

Nach ca, vier Wochen werden gut entwickelte Embryos mit einer deutlichen Polarität (Keimblattstadium, Dos Santos et al., 1983) auf festes 1/2SH-Medium mit 25 $\mu$M (8,6 mg/l) Gibberellinsäure (GA$_3$) und 0,25 $\mu$M (0,046 mg) NAA gesetzt. Nach der Bewurzelung und der Entwicklung eines Sprosses mit Blättern werden die kleinen Pflänzchen auf LS-Medium umgesetzt.

Die Tabelle gibt die Zusammensetzung der Medien B$_5$h, SHJ, SHR, 1/2 SH, LS an. Das flüssige Medium SH entspricht dem SHJ-Medium ohne die Hormone 2,4D und Kinetin. Die Angabe der Mengen ist in mg/l, wenn nichts anderes vermerkt ist.

| Makroelemente | B$_5$h | SHJ | SHR | 1/2SH | LS |
|---|---|---|---|---|---|
| NH$_4$NO$_3$ | - | - | - | | 1650 |
| KNO$_3$ | 3000 | 2500 | 2500 | 1250 | 1900 |
| CaCl$_2$ 2H$_2$O | 895 | 200 | 200 | 100 | 1900 |
| MgSO$_4$ 7H$_2$O | 500 | 400 | 400 | 200 | 370 |
| (NH$_4$)$_2$SO$_4$ | 134 | - | 1651 | - | - |
| NaH$_2$PO$_4$H$_2$O | 156 | - | 407 | - | - |
| KH$_2$PO$_4$H$_2$O | - | - | - | - | 170 |
| NH$_4$H$_2$PO$_4$ | - | 300 | - | 150 | - |

## Mikroelemente

| | B$_5$h | SHJ | SHR | 1/2SH | LS |
|---|---|---|---|---|---|
| ZnSO$_4$H$_2$O | 10 | 10 | 10 | 5 | 22,3 |
| H$_3$BO$_3$ | 3 | 5 | 5 | 2,5 | 6,2 |
| ZnSO$_4$ 7H$_2$O | 1 | 1 | 1 | 0,5 | 8,6 |
| Na$_2$MoO$_4$2H$_2$O | 0,25 | 0,1 | 0,1 | 0,05 | 0,25 |
| CuSO$_4$ 5H$_2$O | 0,025 | 0,02 | 0,02 | 0,1 | 0,025 |
| CaCl$_2$ 6H$_2$O | 0,025 | 0,1 | 0,1 | 0,05 | 0,025 |
| KJ | 0,75 | 1 | 1 | 0,5 | 0,83 |
| FeSO$_4$ 7H$_2$O | 28 | 15 | 15 | 7,5 | 28 |
| Na$_2$EDTA | 37 | 20 | 20 | 10 | 37 |

## Vitamine

| | B$_5$h | SHJ | SHR | 1/2SH | LS |
|---|---|---|---|---|---|
| Thiamin HCl | 10 | 5 | 5 | 2,5 | 0,4 |
| Pyridoxin HCl | 1 | 0,5 | 0,5 | 0,25 | - |
| Nikotinsäure | 1 | 5 | 5 | 2,5 | - |

|  | $B_5h$ | SHJ | SHR | 1/2SH | LS |
|---|---|---|---|---|---|
| **Aminosäuren** | | | | | |
| L-Glutamin | 800 | - | - | - | - |
| L-Serin | 100 | - | - | - | - |
| L-Prolin | - | - | 5755 | - | - |
| **Andere Bestandteile** | | | | | |
| u.a. Phytohormone | | | | | |
| Myo-Inositol | 100 | 1000 | 1000 | 500 | 100 |
| L-Glutathion | 10 | - | - | - | - |
| Adenine-sulfat | 1 | - | - | - | - |
| 2,4-D | 1 | 11,5 | - | - | - |
| Kinetin | 0,2 | 1,075 | - | - | - |
| $GA_3$ | - | - | - | 8,6 | - |
| NAA | - | - | - | 0,046 | - |
| Sucrose | 30 g | 30 g | 30 g | 15 g | 10 g |
| pH | 5,8 | 5,9 | 5,9 | 5,9 | 5,8 |

Die Einstellung des pH-Wertes erfolgte mit 1N KOH.

Die Medien werden durch Erhitzen im Autoklaven für 17 min bei 121°C sterilisiert. Kinetin, L-Glutathion und Aminosäuren werden mit einem Filter sterilisiert und nach dem Erhitzen im Autoklaven dem 60°C warmem Medium zugegeben.

e) Protoplastenkultur

Als Ausgangsmaterial zur Isolierung von Protoplasten dienen die Blätter 2-3 Monate alter steriler Sproßkulturen. Die Ernte erfolgt 2-3 h nach dem Einschalten des Lichtes.

- Zuerst werden die Blätter in einer Petrischale mit EMI (Atanassov und Brown, 1984) befeuchtet und mit einer neuen Rasierklinge fein zerschnitten.
- 1-1,5 g Blätter werden dann mit 10 ml Enzymlösung in einer Petrischale (φ 10 cm) für 3-4 h inkubiert. Die Inkubation erfolgt bei 26°C und schwacher Beleuchtung. Die Freisetzung von Protoplasten aus den Blättern wird unter dem Mikroskop verfolgt. Alle 30 min wird die Schale 2-3 mal geschwenkt.

Die Enzymlösung besteht aus einer Mischung von 1:1 aus dem Protoplastenkulturmedium (AP) von Atanassov und Brown (1984) mit den Hormonen 0,2 mg/l 2,4-D, 0,5 mg/l Zeatin und 1 mg/l NAA und einer Enzymlösung. Die Enzymlösung (Kao und Michayluk, 1979; modifiziert) besteht aus:

- 200 mg Cellulose Onozuka R10
- 80 mg Macerozyme R10      Serva
- 10 mg Pectolyase Y-23      Sigma
- 540 mg Sorbitol

f) Transformation eines induzierten Kallus

Kallus wird nach der Methode, die unter Kallusregeneration beschrieben ist, zur Embryogenese induziert.

Im Anschluß an die 3-4tägige Inkubation in flüssigem SHJ wird das Kallusmaterial auf einem Sieb (Maschenweite 100 $\mu$m) mit flüssigem SHR, das keinen Agar und keinen L-Prolin enthält, gewaschen. Danach wird das Kallusmaterial in flüssigem SHR aufgenommen. Ca. 1 g Kallusmaterial wird 10 ml Medium zugesetzt.

Nach der Zugabe der Agrobakterien, welche Resistenz -Gen tragende Ti-Plasmide enthalten ($2 \times 10^7$/ml Endkonzentration), wird das Kallusmaterial auf einem Schüttler (90Upm) für 2-3 Tage bei 26°C inkubiert.

Danach wird das Material auf einem Sieb (Maschenweite 100 $\mu$m$^2$) mit flüssigem SHR gewaschen.

Die Plattierung (75 mg Kallus/10 ml Medium) erfolgt auf dem normalen festen SHR mit 100 mM L-Prolin. Das Medium in den Platten enthält neben den selektiven Antibiotika 500 $\mu$g/ml Claforan. Nach vier Wochen werden die resistenten Strukturen auf frisches antibiotikahaltiges Medium gesetzt, und weitere drei Wochen später werden sie geteilt und eine Hälfte wird auf frisches Medium ohne selektive Antibiotika, die andere Hälfte auf antibiotikahaltiges Medium gesetzt.

g) Transformation von Embryos

Die Transformation von Embryos wird analog zu der Transformation des induzierten Kallus durchgeführt. Als Ausgangsmaterial werden 4-5 Wochen alte Embryos verwendet. Sie werden in einer Petrischale mit einer Rasierklinge fein zerschnitten und dann auf einem Sieb (Maschenweite 100 $\mu$m) mit flüssigem SHR gewaschen. Ca. 1 g zerschnittene Embryos werden in 10 ml flüssigem SHR aufgenommen. Nach der Zugabe von Agrobakterien ($2 \times 10^7$/ml Endkonzentration) erfolgt die Inkubation auf einem Schüttler (90 Upm) für 2-3 Tage bei 26°C. Danach werden die Embryostücke auf einem Sieb (100 $\mu$m$^2$) mit flüssigem SHR gewaschen. Die Plattierung erfolgt mit einem Spatel auf Platten, die das normale feste SHR mit 100 mM L-Prolin enthalten. Ca. 50-100 mg Embryostücke werden pro Platte, die 10 ml Medium enthält, verteilt. Das Medium in den Platten enthält neben den selektiven Antibiotika 500 $\mu$g/ml Claforan. Drei Wochen nach der Plattierung werden die sekundären Embryos auf frischen Platten subkultiviert. Gut entwickelte Embryos werden auf antibiotikafreies 1/2 SH-Medium (Stuart und Strickland, 1984, b) gesetzt, um eine Weiterentwicklung zu Pflanzen zu ermöglichen. Kleine Pflänzchen mit Wurzeln werden dann auf LS Medium umgesetzt.

4. Transformation von Solanum tuberosum (Kartoffel)

Die Transformation wird genau nach dem in der EP-A-0 242 246, Seiten 14 bis 15 angegebenen Weise durchgeführt, wobei die Agrobakterien Ti-Plasmide enthalten, die das Resistenz-Gen tragen.

Alle Prozentangaben in den obigen Beispielen beziehen sich auf Gewichtsprozente, wo nichts anderes angegeben wird.

In den gemäß den obigen Beispielen erhaltenen transgenen Pflanzenzellen und Pflanzen kann die Übertragung und die Ausprägung der Resistenz-Gene mit Hilfe zweier Kriterien nach den allgemein üblichen Methoden eindeutig nachgewiesen werden. So sind die Gene und ihre primären Genprodukte mit Hilfe der oben erwähnten Probe eindeutig nachweisbar. Weiterhin werden die primären Genprodukte präferentiell dann ausgeprägt, wenn die transgenen Pflanzen mit einem Pathogen, z.B. durch Phytophthora megasperma oder dessen Zellwandfragmente infiziert wurden. Die biologische Wirkung im Sinne einer erhöhten Resistenz gegen Schadorganismen kann durch vermindertes Wachstum der Pathogene und durch einen reduzierten Pflanzenschaden nachgewiesen werden.

Im folgenden werden einige der bei der Transformation von Pflanzen bzw. Pflanzenzellen eingesetzte Medien beschrieben:

| Am-Medium | |
|---|---|
| 3,5 g | $K_2HPO_4$ |
| 1,5 g | $KH_2PO_4$ |
| 0,5 g | $Na_3$ Citrat |
| 0,1 g | $MgSO_4 \times 7H_2O$ |
| 1 g | $(NH_4)_2SO_4$ |
| 2 g | Glukose ad 1 l |

Medium für sterile Sproßkultur von Tabak

Macro-elemente 1/2 der Konzentration der MS Salze
Micro-elemente 1/2 der Konzentration der MS Salze

```
Fe-EDTA              Murashige und Skoog (MS)


Myo-Inosit                                  100   mg/l
Sucrose                                      10   mg/l
Agar                                          8   g/l
Vitamine    Ca-pantothenat                    1   mg/l
            Biotin                           10   mg/l
            Nicotinsäure                      1   mg/l
            Pyridoxin                         1   mg/l
            Thiamin                           1   mg/l


pH 5,7 vor dem Autoklavieren
```

K3-Medium

Zur Kultur von Nicotiana tabacum petit Havana SR1, Nicotiana tabacum Wisconsin 38, und Nicotiana plumbaginifolia Protoplasten (Nagy und Maliga, 1976)

| Macro-elemente | $NH_4NO_3$ | 250 | mg/l |
|---|---|---|---|
| | $KNO_3$ | 2500 | mg/l |
| | $CaCl_2 \cdot 2H_2O$ | 900 | mg/l |
| | $MgSO_4 \cdot 7H_2O$ | 250 | mg/l |
| | $NaH_2PO_4 \cdot 1H_2O$ | 150 | mg/l |
| | $(NH_4)_2SO_4$ | 134 | mg/l |
| | $CaHPO_4 \cdot 1H_2O$ | 50 | mg/l |
| Micro-elemente | $H_3BO_3$ | 3 | mg/l |
| | $MnSO_4 \cdot 1H_2O$ | 10 | mg/l |
| | $ZnSO_4 \cdot 4H_2O$ | 2 | mg/l |
| | $KI$ | 0,75 | mg/l |
| | $Na_2MoO_4 \cdot 2H_2O$ | 0,25 | mg/l |
| | $CuSO_4 \cdot 5H_2O$ | 0,025 | mg/l |
| | $CoCl_2 \cdot 6H_2O$ | 0,025 | mg/l |
| Fe-EDTA | $Na_2EDTA$ | 37,2 | mg/l |
| | $FeSO_4 \cdot 7H_2O$ | 27,8 | mg/l |
| Inosit | | 100 | mg/l |
| Sucrose | | 137 | g/l |
| | | (= 0,4 M) | |
| Xylose | | 250 | mg/l |
| Vitamine | Nicotinsäure | 1 | mg/l |
| | Pyridoxin | 1 | mg/l |
| | Thiamin | 10 | mg/l |
| Hormone | NAA | 1,0 | mg/l |
| | Kinetin | 0,2 | mg/l |

pH 5,6

Filter sterilisieren

Linsmaier und Skoog Medium (Linsmaier und Skoog 1965)

Zur Kultur von regenerierenden Protoplasten und für Gewebekultur von Tabaktumoren und Kallus. Linsmaier und Skoog (LS) Medium ist Murashige und Skoog Medium (Murashige und Skoog, 1962) mit den folgenden Modifikationen:
- Thiamin wird in höherer Konzentration eingewogen 0,4 mg/l anstatt 0,1 mg/l;
- Glycin, Pyridoxin und Nicotinsäure fehlen.

| Macro-elemente | $NH_4NO_3$ | 1650 | mg/l |
|---|---|---|---|
| | $KNO_3$ | 1900 | mg/l |
| | $CaCl_2.2H_2O$ | 440 | mg/l |
| | $MgSO_4.7H_2O$ | 370 | mg/l |
| | $KH_2PO_4$ | 170 | mg/l |
| Micro-elemente | $H_3BO_3$ | 6,2 | mg/l |
| | $MnSO_4.1H_2O$ | 22,3 | mg/l |
| | $ZnSO_4.4H_2O$ | 8,6 | mg/l |
| | $KI$ | 0,83 | mg/l |
| | $Na_2MoO_4.2H_2O$ | 0,25 | mg/l |
| | $CuSO_4.5H_2O$ | 0,025 | mg/l |
| | $CoCl_2.6H_2O$ | 0,025 | mg/l |
| Fe-EDTA | $Na_2EDTA$ | 37,2 | mg/l |
| | $FeSO_4.7H_2O$ | 27,8 | mg/l |
| Inosit | | 100 | mg/l |
| Saccharose | | 30 | g/l |
| Agar | | 8 | g/l |
| Vitamine | Thiamin | 0,4 | mg/l |
| Hormone: | NAA | 1 | mg/l |
| | Kinetin | 0,2 | mg/l |

**pH 5,7 vor dem Autoklavieren**

Zur Transformation von Pflanzen bzw. Pflanzenzellen sowie bezüglich Resistenz-Genen kann zur weiteren Erläuterung der eingesetzten Verfahren und Methoden die folgende Literatur angeführt werden:

Aerts M, Jacobs M, Hernalsteens JP, Van Montagu M, Schell J (1983) Induction and in vitro culture of Arabidopsis thaliana crown gall tumours. Plant Sci Lett. 17: 43-50

Atanasov A, Brown DCW (1984) Plant regeneration from suspension culture and mesophyll protoplasts of Medicago sativa L. Plant Cell Tiss Org. Cult. 3, 149-162

Davey MR, Cocking EC, Freeman J, Pearce N, Tudor I (1980) Transformation of Petunia protoplasts by isolated Agrobacterium plasmid. Plant Sci Lett 18: 307-313

Deblaere R., Bytebier B., De Greve H, Deboeck F, Schell J, van Montagu M, Leemans J (1985) Efficient octopine Ti plasmid-derived vectors for Agrobacterium-mediated gene transfer to plants. Nucleic Acid Research, Vol. 13, No. 13, 4777 (1985)

Fraley R.T. et al., Proc. National Acad. Sci USA 80, 4803 (1983)

Fromm ME, Taylor LP, Walbot V (1986) Stable transformation of maize after gene transfer by electroporation. Nature 319: 791-793

Gamborg O.L., Miller R.A. and Ojiima K. (1968) Nutrient requirements of suspension cultures of soybean root cells, Experimental Cell Research 50: 151-158

Hain, R., Stabel, P., Czernilofsky, A.Pp., Steinbiß, H.H., Herrera-Estrella, L., Schell, J. (1985) Uptake, integration, expression and genetic transmission of a selectable chimeric gene by plant protoplasts. Molec Gen Genet 199: 161-168

Herrera-Estrella L., De Block M., Messens E., Hernalsteens JP., van Montagu M., Schell J. (1983) EMBO J. 2: 987-995.

Horsch RB, Fry JE, Hoffmann NL, Eichholtz D, Rogers SG, Fraley RT (1985) A simple and general method for transferring genes into plants. Science 277: 1229-1231

KaO KN, Michayluk MR (1980) Plant regeneration from Mesophyll protoplasts of Alfalfa. Z Pflanzenphysiol. 96, 135-141

Keller WA, Melchers G (1973) The effect of high pH and calcium on tobacco leaf protoplast fusion. Z Naturforschg 28c: 737-741

Krens FH, Molendijk L, Wullems GJ, Schilperoort RA (1982) in vitro transformation of plant protoplasts with Ti-plasmid DNA. Nature 296: 72-74 Koncz C, Schell J (1986) The promotor of $T_L$-DNA gene 5 controls the tissue-specific expression of chimaeric genes carried by a novel type of Agrobacterium linary vector. Mol. Gen. Genet. (1986) 204: 338-396

Linsmaier DM, Skoog F (1965) Organic growth factor requirements of tobacco tissue cultures. Physiol Plant 18: 100-127

Marton L, Wullems GJ, Molendijk L, Schilperoort PR (1979) In vitro transformation of cultured cells from Nicotiana tabacum by Agrobacterium tumefaciens. Nature 277: 1229-131

Nagy JI, Maliga P (1976) Callus induction and plant regeneration from mesophyll protoplasts of Nicotiana sylvestris. Z Pflanzenphysiol 78: 453-455

Oelck Michael M., Dissertation, 1984, University of Cologne, Federal Republic of Germany, Regeneration von Leguminosen aus Gewebe- und Zellkulturen

Otten LABM, Schilperoort RA (1978) A rapid microscale method for the detection of Lysopin and Nopalin dehydrogenase activities. Biochim biophys acta 527: 497-500

Paszkowski J, Shillito RD, Saul M, Mandak V, Hohn T, Hohn B, Potrykus I (1984) Direct gene transfer to plants. EMBO J 3: 2717-2722

Rolfs, Fritzemeier, Kindl (1981) Plant Cell Reports 1:83-85

Reiss Bernd, Sprengel Rolf, Will Hans and Schaller Heinz (1984) A new sensitive method for qualitative and quantitative assay of neomycin phosphotransferase in crude cell tracts, GENE 1081: 211-217

Sambrook, Fritsch und Maniatis, 1989, Molecular Cloning; A Laboratory Manual 2nd Ed., Cold Spring Harbor Laboratory Press USA

Schreier Peter H., Seftor Elisabeth A., Schell Jozef and Bohnert Hans J. (1985) The use of nuclear-encoded sequences to direct the light-regulated synthesis and transport of a foreign protein into plant chloroplasts, EMBO J Vol. 4, No. 1: 25-32

Shenk RU, Hildebrandt AC (1972) Medium and techniques for induction and growth of monocotyledonous and dicotyledonous cell cultures. Ca. J. Bot. 50, 199-204

Shillito RD, Paszkowski J. Potrykus I (1983) Agarose plating and Bead type culture technique enable and stimulate development of protoplast-derived colonies in an number of plant species. Pl Cell Rep 2: 244-247 Simons-Schreier A, Dissertation Universität Köln (1988) Studien zur Transformation von Medicago sativa und zur Expression eines Leghämoglobingens (1bc3) und eines chimären 1b-cat-Gens während der Symbiose in transgenen Pflanzen

Stuart DA, Strickland SG (1984) Somatic embryogenesis from cell cultures of Medicago sativa L. I. The role of amino acid additions to the regeneration medium. Plant Sci. Let. 34, 165-174

Stuart DA, Strickland SG (1984) Somatic embryogenesis from cell cultures of Medicago sativa L. II. The interaction of amino acids with ammonium. Plant Sci. Let. 34, 175-181

Van den Elzen PJM, Townsend J, Lee KY, Bedbrook JR (1985) Achimaeric resistance gen as a selectable marker in plant cells. Plant Mol. Biol. 5, 299-302.

Velten J, Velten L, Hain R, Schell J (1984) Isolation of a dual plant promotor fragment from the Ti Plasmid of Agrobacterium tumefaciens. EMBO J 12: 2723-2730

Walker KA, Yu PC, Sato SJ, Jaworski EG (1978) The hormonal control of organ formation in callus of Medicago sativa L. cultured in vitro. Amer. J. Bot. 65(6), 654-659

Walker KA, Sato SJ (1981) Morphogenesis in callus tissue of Medicago sativa: The role of ammonium ion in somatic ambryogenesis. Plant Cell Tiss. Org. Cult. 1, 109-121

Wullems GJ, Molendijk L, Ooms G, Schilperoort RA (1981) Differential expression of crown gall tumor markers in transformants obtained after in vitro Agrobacterium tumefaciens - induced transformation of cell wall regenerating protoplasts derived from Nicotiana tabacum. Proc Natl Acad Sci 78: 4344-4348 Yanisch-Perron, C., Vicira, J. und Messing, J. (1985) Gene 33, 103-119

Zambryski P, Joos H, Genetello C, van Montagu M, Schell J (1983) Ti-plasmid vector for the introduction of DNA into plant cells without altering their normal regeneration capacity, EMBO J 12: 2143-2150.

Weiterhin können die folgenden veröffentlichten Patentanmeldungen aufgeführt werden:

EP-A 116 718

EP-A 159 418

EP-A 120 515

EP-A-120 516

EP-A-172 112
EP-A-140 556
EP-A-174 166
EP-A-122 791
EP-A-126 546
EP-A-164 597
EP-A-175 966
EP-A-270 822
WO 84/02913
WO 84/02919
WO 84/02920
WO 83/01176

**Patentansprüche**

1. Pflanzliche Resistenz-Gene, welche dadurch gekennzeichnet sind, daß sie spezifisch durch Pathogene induzierbar sind und DNA-Sequenzen enthalten, die der cDNA der folgenden Sequenz entsprechen:

```
1                                                    50
ATCTCGTTCA AGTCGGCGCT CTGGTTGTGG ATGACAGAGC AGAAACCAAA

51                                                  100
ACCTTCTTGC CACAACGTCA TGGTTGGGAA TTACGTGCCA ACAGCATCTG

101                                                 150
ATAGAGCAGC AAATAGAACC TTAGGGTTTG GGTTGGTTAC GAACATCATC

151             170
AACGGCGGCC TGGACTGCGG
```

2. Resistenz-Gene gemäß Anspruch 1, welche für Hydrolasen kodieren.

3. Resistenz-Gene gemäß Anspruch 1, welche aus Arachis hypogaea erhältlich sind.

4. cDNA der Resistenz-Gene gemäß den Ansprüchen 1 bis 3.

5. Regulatorisch wirkende Teile der Resistenz-Gene gemäß den Ansprüchen 1 bis 3.

6. Struktur-Gene der Resistenz-Gene gemäß den Ansprüchen 1 bis 3.

7. DNA der Sequenz

```
1                                                    50
ATCTCGTTCA AGTCGGCGCT CTGGTTGTGG ATGACAGAGC AGAAACCAAA

51                                                  100
ACCTTCTTGC CACAACGTCA TGGTTGGGAA TTACGTGCCA ACAGCATCTG

101                                                 150
ATAGAGCAGC AAATAGAACC TTAGGGTTTG GGTTGGTTAC GAACATCATC

151             170
AACGGCGGCC TGGACTGCGG
```

**8.** Rekombinante prokaryontische oder eukaryontische DNA, welche ein oder mehrere Resistenz-Gene bzw. Gen-Einheiten und/oder ihre Teile und/oder die DNA gemäß den Ansprüchen 1 bis 7 als "fremde" DNA oder als "zusätzliche" DNA enthält.

**9.** Rekombinante DNA gemäß Anspruch 8, welche in Pflanzenzellen (einschließlich Protoplasten) oder Pflanzen (einschließlich Pflanzenteilen und Samen) enthalten ist.

**10.** Vektoren, welche ein oder mehrere Resistenz-Gene bzw. Gen-Einheiten oder ihre Teile und/oder die DNA gemäß den Ansprüchen 1 bis 7 und/oder rekombinante DNA gemäß Anspruch 8 enthalten.

**11.** Plasmid pR 3-7.

**12.** Transformierte Mikroorganismen, welche ein oder mehrere Resistenz-Gene bzw. Gen-Einheiten und/oder ihre Teile und/oder die DNA gemäß den Ansprüchen 1 bis 7 und/oder rekombinante DNA gemäß Anspruch 8 enthalten.

**13.** Escherichia coli Stamm RG-2 (DSM 6149) und seine Mutanten.

**14.** Verwendung der Resistenz-Gene und/oder ihrer Teile und/oder der DNA und/oder der Vektoren und/oder der transformierten Mikroorganismen gemäß den Ansprüchen 1 bis 13 zur Transformation von Pflanzenzellen (einschließlich Protoplasten) und Pflanzen (einschließlich Pflanzenteilen und Samen).

**15.** Transgene Pflanzenzellen (einschließlich Protoplasten) und Pflanzen (einschließlich Pflanzenteilen und Samen), welche ein oder mehrere Resistenz-Gene und/oder deren Teile und/oder die DNA gemäß den Ansprüchen 1 bis 8 als "fremde" oder "zusätzliche" DNA enthalten.

**16.** Verfahren zur Herstellung transgener Pflanzenzellen (einschließlich Protoplasten) und Pflanzen (einschließlich Pflanzenteile und Samen) gemäß Anspruch 15, dadurch gekennzeichnet, daß man
(a) ein oder mehrere Resistenz-Gene und/oder ihre Teile und/oder die DNA gemäß den Ansprüchen 1 bis 8 in das Genom von Pflanzenzellen (einschließlich Protoplasten) einsetzt und gegebenenfalls
(b) aus den transformierten Pflanzenzellen (einschließlich Protoplasten) vollständige transgene Pflanzen regeneriert und gegebenenfalls
(c) von den so erhaltenen transgenen Pflanzen der Elterngeneration oder weiterer daraus gewonnener Generationen die gewünschten Pflanzenteile (einschließlich Samen) gewinnt.

**17.** Verwendung von transgenen Pflanzenzellen (einschließlich Protoplasten) und Pflanzen (einschließlich Pflanzenteilen und Samen) gemäß Anspruch 15 zur Erzeugung von Vermehrungsmaterial sowie zur Erzeugung neuer Pflanzen und deren Vermehrungsmaterial.

**18.** Vermehrungsmaterial, erhältlich durch die Vermehrung der transformierten Pflanzenzellen und Pflanzen gemäß Anspruch 15.

**19.** Verwendung der DNA gemäß Anspruch 7 als Sonde beim Auffinden, Isolieren, Reinigen und/oder Nachweisen der erfindungsgemäßen Resistenz-Gene und/oder ihrer Teile.

**20.** Pflanzliche Resistenz-Gene, die spezifisch durch Pathogene induzierbar sind und mit Hilfe der cDNA gemäß Anspruch 7 als Sonde isolierbar sind.